# EUROPEAN PATENT APPLICATION

(11) **EP 3 919 497 A1**
(43) Date of publication of application: **08.12.2021**
(21) Application number: 20460027.4
(22) Date of filing: 04.06.2020
(51) Int. Cl.: C07D 487/04

(54) **PROCESS FOR THE PREPARATION OF TICAGRELOR**

(71) Applicant: Zaklady Farmaceutyczne Polpharma S.A., 83-200 Starogard Gdanski (PL)
(72) Inventor: SZEPINSKI, Emil, 83-200 Starogard Gdanski (PL); KOSIK, Kamil, 83-200 Starogard Gdanski (PL); SAGOL, Karol, 83-110 Tczew (PL)
(74) Representative: Kondrat, Mariusz

(57) **Abstract**

The present invention relates to a process for the preparation of ticagrelor, which provides a product of high purity, in particular, with no detectable levels of N-nitrosmine impurities. The process comprises a first step of treating 2-[[(3aR,4S,6R,6aS)-6-[[5-amino-6-chloro-2-(propylthio)-4-pyrimidinyl]amino]tetrahydro-2,2-dimethyl-4H-cyclopenta-1,3-dioxol-4-yl]oxy]ethanol starting material with sodium nitrite, followed by acidic washing; in a second step, the 2-[[(3aR,4S,6R,6aS)-6-[7-chloro-5-(propylthio)-3H-1,2,3-triazolo[4,5-d]pyrimidin-3-yl]tetrahydro-2,2-dimethyl-4H-cyclopenta-1,3-dioxol-4-yl]oxy]ethanol obtained in the previous step is coupled with trans-(1R,2S)-2-(3,4-difluorophenyl)cyclopropylamine, and the reaction is followed by a first washing at basic pH a second washing at acidic pH; and in an third step, the compound obtained in the previous step is deprotected by treatment with mineral acid, followed by acidic washing.

## Description

### Technical field

The present invention relates to a new process for the preparation of the antiplatelet drug ticagrelor.

### State of the art

Ticagrelor [(1*S*,2*S*,3*R*,5*S*)-3-[7-{[(1*R*,2*S*)-2-(3,4-difluorophenyl) cyclopropyl]amino}-5-(propylsulfanyl)-3*H*-[1,2,3]triazolo[4,5-*d*]pyrimidin-3-yl]-5-(2-hydroxyethoxy)cyclopentane-1,2-diol] is a platelet aggregation inhibitor, specifically, it is a potent antagonist of the platelet P2Y₁₂ receptor that prevents ADP-mediated P2Y₁₂ dependent platelet activation and aggregation.

Ticagrelor, which is marketed under the trade names Brilique and Brilinta, is used in therapy as an oral drug, for the prevention of thrombotic cardiovascular events in patients with acute coronary syndromes.

Ticagrelor was first disclosed in the international patent application WO00/34283-A1.

Chemically, ticagrelor is a nucleoside analogue and belongs to chemical class cyclopentyl-triazolo-pyrimidines (CPTP). The chemical structure of ticagrelor is depicted below:

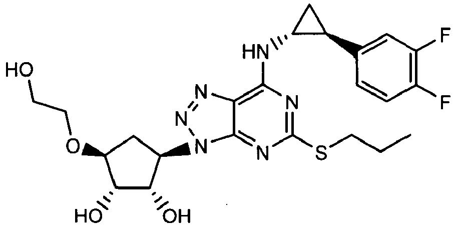

The international patent application WO01/92263-A1 discloses the preparation of an intermediate for the synthesis of ticagrelor (compound of Formula I) and its use for preparing ticagrelor.

The process disclosed in WO01/92263-A1 for the synthesis of ticagrelor starting from this intermediate is shown in Scheme I. Thus, the intermediate of Formula I first undergoes cyclization by treatment with sodium nitrite in the presence of acetic acid, the formed product is then coupled with the mandelate salt of trans-(1*R*,2*S*)-2-(3,4-difluorophenyl)cyclopropylamine in the presence of triethylamine to provide protected ticagrelor, which is subsequently deprotected by treatment with aqueous hydrochloric acid in methanol to furnish ticagrelor:

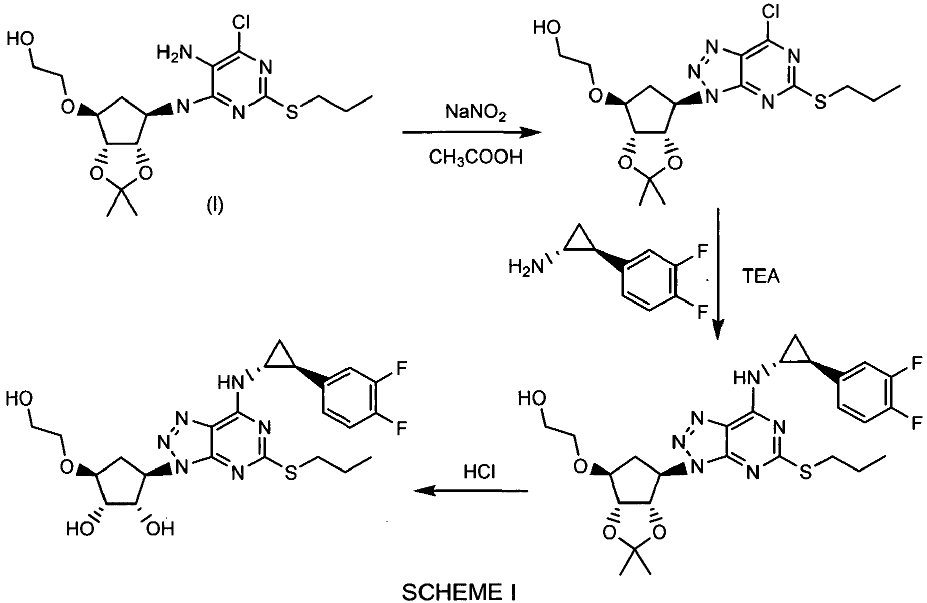

The preparation of said intermediate compound of Formula I is also described in WO01/92263-A1 according to the following reaction, which takes place in the presence of trimethylamine (TEA):

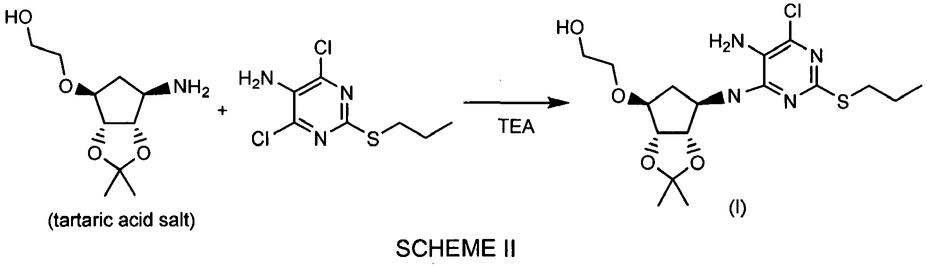

There is a general concern about the presence of nitrosamines as impurities in synthetic active pharmaceutical ingredients (APIs) (*Information on nitrosamines for marketing authorisation holders,* EMA/189634/2019). Nitrosamine impurities are worrying because these substances are probable human carcinogens. Among the nitrosamines that can potentially be present as impurities in APIs are, for example, *N-*nitrosodimethylamine (NDMA), *N*-nitrosodiethylamine (NDEA), *N-*nitrosoethylisopropylamine (NEIPA) and *N*-nitrosodiisopropylamine (NDIPA).

The use of sodium nitrite (NaNO₂), or other nitrites, in the presence of secondary or tertiary amines is a potential cause of nitrosamine formation.

The process for preparing ticagrelor disclosed in WO01/92263-A1 has the disadvantage of giving rise to nitrosamine impurities, due to the use of sodium nitrite and tertiary amines (triethylamine).

Even if tertiary amines are avoided in the preparation of ticagrelor from the compound of Formula I, there is still the risk that nitrosamines are formed due to the possible presence of tertiary amines as impurities in the starting materials and/or reactants.

Therefore, there is the need to provide an improved method for the preparation of ticagrelor that can afford this substance in good yield and with low levels of impurities, in particular, of nitrosamine impurities.

### Object of the invention

The object of the present invention is a process for the preparation of ticagrelor.

### Description of the Drawings

Figure 1 shows the X-ray powder diffraction (XRPD) of ticagrelor obtained in Example 1. The x-axis shows the 2theta angle (in degrees) and the y-axis shows the intensity.
Figure 2 shows the differential scanning calorimetry (DSC) thermogram of ticagrelor obtained in Example 1.

### Detailed description of the invention

The object of the present invention is a process for the preparation of ticagrelor of formula: 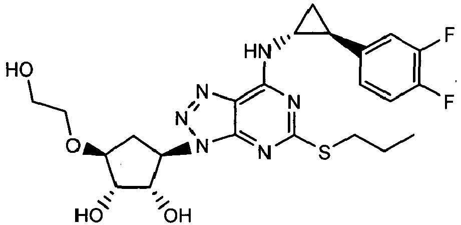 comprising the following steps:
a) reacting a compound of formula (I): 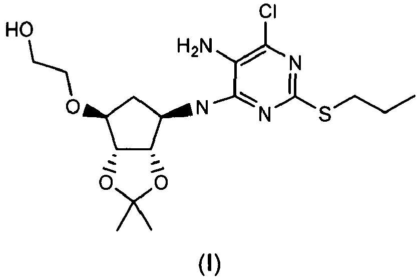 with sodium nitrite, in the presence of acetic acid, to provide a compound of formula (II): 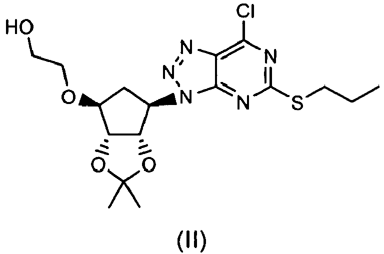 , wherein the reaction is followed by aqueous washing at a pH comprised in the range 2-6;
b) reacting a compound of formula (II) with a compound of formula (III): 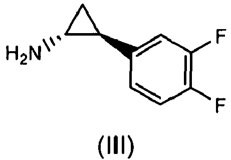 or a pharmaceutically acceptable salt thereof, in an organic solvent, in the presence of a base, to provide a compound of formula (IV): 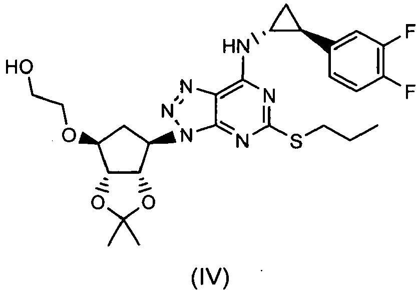 , wherein the reaction is followed by a first aqueous washing at a pH comprised in the range 7.5-12 and a second aqueous washing at a pH comprised in the range 2-6; and
c) deprotecting a compound of formula (IV) by treatment with an inorganic acid in a solvent mixture of water and a C₁-C₃ alcohol to provide ticagrelor of formula (I), wherein the deprotection step is followed by addition of an organic solvent and aqueous washing at a pH comprised in the range 0.5-6.0.

The authors of the present invention have surprisingly found that the preparation of ticagrelor according to the above process provides good yield of the final product and undetectable nitrosamine impurities, despite the fact of using sodium nitrite and tertiary amines in the process.

Without wishing to be bound to any theory, it has been found that by controlling the pH after each reaction, in particular, in the successive washings performed after each step, it is possible to significantly reduce the presence of nitrosamine impurities, to no detectable levels.

As shown in Example 2, the process of the present invention is very efficient for avoiding nitrosamine impurities in the final product, even when a tertiary amine is used in the process, and is also effective to suppress nitrosamine impurities in less demanding conditions, when a tertiary or secondary amine might not be used in the process but may be present anyway in the reaction mixture, as impurities, for example, of the compound of formula (I) or of other intermediates and/or reactants.

Along the present description, as well as in the claims, the singular expressions, generally preceded by the articles "a", "an" or "the", are meant to include also the plural forms, unless the context clearly indicates otherwise. Furthermore, numeric values preceded by the term "about" are meant to include the exact stated value and also a certain variation around such value, namely a variation or ±5% of the stated amount. Numeric ranges defined by lower and upper endpoints are meant to include also said stated endpoints. The percentages disclosed (%, wt%) are always weight percentages, unless otherwise specified.

As used herein, aqueous washing (or aqueous workup) is a purification step to remove water-soluble impurities from a reaction medium comprising a mixture of immiscible organic and aqueous solvents, so phases are separated and the aqueous phase is discarded. Aqueous washing frequently involves the addition of water or an aqueous solution to a reaction medium comprising an organic solvent, followed by separation of phases and discarding the aqueous phase. Or, conversely, aqueous washing may involve adding an organic solvent to a reaction medium comprising water or an aqueous solution, also followed by separation of phases and discarding the aqueous phase. The aqueous phase may also contain water miscible organic solvents, typically alcohols, such as methanol, if present in the reaction mixture.

The aqueous washing also typically involves pH regulation before separation of phases. This pH regulation, as performed in the present invention, may be related to the good yield and low nitrosamine content of the present process. pH adjustments, if needed, may be done, typically, with aqueous sodium hydroxide or with aqueous hydrochloric acid, for example.

The volume of water or aqueous solution to be used in the washings may be readily adjusted by the skilled in the art according to the normal practice in organic reaction procedures. For example, the volume of the organic phase relative to the aqueous phase may be in the range 1:0.5 to 1:3, preferably in the range 1:0.75 to 1:2.

In one embodiment the aqueous washing steps performed with water are performed with purified water, also known as demineralized water. Purified water in the context of the present invention means European Pharmacopeia grade purified water.

In some cases, as is well-known in the art, aqueous high-concentration solution of sodium chloride (brine) or another similar salt (such as potassium chloride, for example) may be used for the washing steps. Typically, brine, as used herein, is a 15% sodium chloride aqueous solution, but other concentrations of sodium chloride (or other similar salts) may be suitable as well. The use of brine, instead of water, has generally the advantage of avoiding emulsion formation during workup.

In one embodiment, the process of the invention is performed under nitrogen atmosphere.

### First step

The first step of the process of the present invention (step a)) comprises reacting a compound of formula (I): 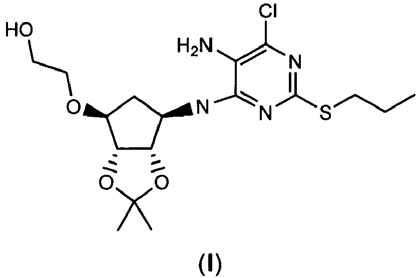 with sodium nitrite, in the presence of acetic acid to provide a compound of formula (II): 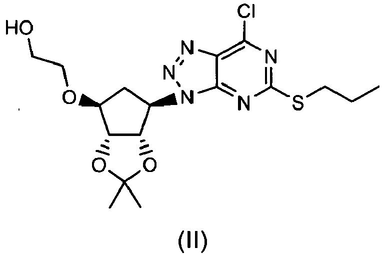 , wherein the reaction is followed by aqueous washing at a pH comprised in the range 2-6.

The starting compound of formula I, which can be named as 2-[[(3aR,4S,6R,6aS)-6-[[5-amino-6-chloro-2-(propylthio)-4-pyrimidinyl]amino]tetrahydro-2,2-dimethyl-4H-cyclopenta-1,3-dioxol-4-yl]oxy]ethanol can be obtained, for example, as described in the international patent application WO01/92263-A1.

The molar ratio of NaNO₂ to the compound of formula I in this reaction is typically comprised between 1.5:1 and 1:1, preferably comprised between 1.3:1 and 1:1, and more preferably is about 1.1:1.

The molar ratio of acetic acid to the compound of formula I in this reaction is typically comprised between 1:0.05 and 1:0.5, preferably comprised between 1:0.1 and 1:0.25.

In one embodiment, the reaction takes place in aqueous environment, formed by water and acetic acid. In this case, an organic solvent is added afterwards for workup.

In another embodiment, the reaction takes place in a two-phase-system comprising water, acetic acid and an organic solvent.

The volume ratio of the organic solvent (either if added at the beginning of the reaction or later, for workup) to aqueous phase is typically comprised between 20:1 and 1:1, preferably comprised between 10:1 and 4:1.

Suitable organic solvents are, for example, dichloromethane (DCM), ethyl acetate, chloroform, tetrahydrofuran (THF) or methyl *tert*-butyl ether (MTBE), among others.

In one embodiment, the solvent used in step a) is dichloromethane.

Sodium nitrite is typically dissolved in water, previously to be added.

The temperature during the reaction is typically held under 20°C, and preferably is comprised between -10°C and 10°C, preferably comprised between 0°C and 10°C, and more preferably comprised between 0°C and 5°C.

The reaction time is generally comprised between 15 minutes and 10 hours, preferably comprised between 30 minutes and 4 hours, typically under stirring.

The reaction is followed by aqueous washing at acidic pH, ranging from 2 to 6. This washing is typically performed in the same acidic conditions at which the reaction has taken place, without the need of pH adjustment.

For workup, before phase separation, organic solvent may be added (e.g. in case that the reaction has taken place in aqueous conditions) and/or water or brine (i.e. about 15% NaCl aqueous solution) may be added (e.g. in case that the reaction has taken place in two-phase system).

In one embodiment, after the first acidic aqueous washing, a second aqueous washing is performed in basic pH conditions, typically adjusting the pH to a value in the range 7.0-11.0, preferably in the range 8.0-9.5, and still more preferably in the range 8.5-9.0. This second aqueous washing may be performed either with water or with brine, preferably with brine, and the pH adjustment is done with a suitable base, for example with sodium hydroxide solution.

Further aqueous washings may be optionally performed, using water or with brine.

After the washings, the compound of formula (II) (2-[[(3aR,4S,6R,6aS)-6-[7-chloro-5-(propylthio)-3H-1,2,3-triazolo[4,5-d]pyrimidin-3-yl]tetrahydro-2,2-dimethyl-4H-cyclopenta-1,3-dioxol-4-yl]oxy]ethanol) is obtained, as a solution in said organic solvent. The organic solvent can be distilled, to obtain the compound of formula (II) as a residue or, alternatively, the solution can be directly used, without solvent removal, in the following step.

### Second step

The second step of the process of the present invention (step b)) comprises reacting a compound of formula (II), obtained in the first step, with a compound of formula (III): 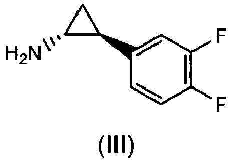 or a pharmaceutically acceptable salt thereof, in an organic solvent, in the presence of a base, to provide a compound of formula (IV): 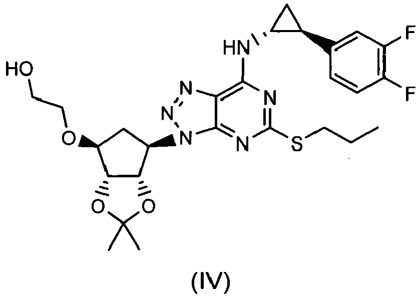 , wherein the reaction is followed by a first aqueous washing at a pH comprised in the range 7.5-12 and a second aqueous washing at a pH comprised in the range 2-6.

The compound of formula (II) can be first dissolved in a suitable organic solvent, which can be selected, for example, from dichloromethane (DCM), ethyl acetate, chloroform, tetrahydrofuran (THF) and methyl *tert*-butyl ether (MTBE), among others.

In one embodiment, the same solvent as in step a) is used.

In one embodiment, the solvent is dichloromethane.

In one embodiment, the solution of the compound of formula (II) obtained in step a) is directly used in step b), without solvent removal.

In one embodiment, about equimolar amount of the compound of formula (III) (trans-(1*R*,2*S*)-2-(3,4-difluorophenyl)cyclopropylamine), or a pharmaceutically acceptable salt thereof, relative to the compound of formula (II) is used.

The reaction is performed in the presence of a base.

In one embodiment, the base is an inorganic base, for example, sodium carbonate or potassium carbonate, among others, typically used in an aqueous solution.

In another embodiment, the base is an organic base, namely an amine, for example, a cyclic amine, such as pyridine, pyrimidine, *N*-methylmorpholine or 1,4-diazabicyclo[2.2.2]octane (DABCO), among others, or a tertiary amine such as *N,N-*dimethylaniline, *N,N*-diethylaniline, triethylamine (TEA), *N,N*-diethylisopropylamine, *N,N-*diisopropylethylamine (DIPEA), tripropylamine or tributylamine, among others.

In one embodiment, the amine is a tertiary amine. Preferably, the amine is selected from triethylamine and *N,N-*diisopropylethylamine, and more preferably is *N,N*-diisopropylethylamine.

The molar ratio of the base to the compound of formula (II) in this reaction is generally comprised between 3:1 and 0.9:1. Higher amount of base can also be used, but is generally not necessary.

The amount of base needed for the reaction may be dependent on whether the compound of formula (III) is used as free base or as a pharmaceutically acceptable salt thereof. When it is used as a salt, one additional mole of base may be required for neutralizing the accompanying acid, while this additional amount of base would not be necessary when the compound of formula (III) is used as a free base.

In one embodiment, the molar ratio of the base to the compound of formula (II) is comprised between 1.9:1 and 2.5:1.

In another embodiment, the molar ratio of the base to the compound of formula (II) is comprised between 0.9:1 and 1.5:1.

The temperature during the reaction is typically comprised between 15°C and 35°C, preferably comprised between 20°C and 25°C.

The reaction time is generally comprised between about 30 minutes and about 20 hours, preferably comprised between about 2 hours and about 10 hours.

The reaction is followed by a first aqueous washing at a pH comprised in the range 7.5-12 and a second aqueous washing at a pH comprised in the range 2-6.

The first washing is generally done by adding water to the reaction media, and the basic pH (7.5-12) is typically that of the reaction media, so pH adjustment may not be required. The pH in this first washing is preferably comprised in the range 8-10.

The second washing is in acidic pH, namely, the pH is adjusted to the range 2-6, preferably to the range 3-5, by adding water and adjusting the pH with aqueous hydrochloric acid.

Further aqueous washings may be optionally performed, typically by adding water and further separation of phases.

In one embodiment, after the washings, the solvent is distilled off under reduced pressure.

### Third step

The third step of the process of the present invention (step c)) comprises deprotecting a compound of formula (IV) by treatment with an inorganic acid in a solvent mixture of water and a C₁-C₃ alcohol to provide ticagrelor of formula (I), wherein the deprotection step is followed by addition of an organic solvent and aqueous washing at a pH comprised in the range 0.5-6.0.

Typically, a solution of the compound of formula (IV) obtained in step b) is prepared in a solvent mixture consisting of water and a C₁-C₃ alcohol, and an inorganic acid is added thereto to perform the deprotection reaction.

The volume ratio water:alcohol in the solvent mixture is not critical, and is typically comprised in the range 1:5 to 1:1, for example about 1:3.5.

The C₁-C₃ alcohol is selected from methanol, ethanol, propanol and isopropanol, preferably is selected from methanol, ethanol and isopropanol, and more preferably is methanol.

The inorganic acid is typically selected from sulphuric acid, phosphoric acid and hydrochloric acid. Preferably hydrochloric acid is used, for example, concentrated hydrochloric acid (aqueous solution of about 35 wt%) can be used.

The temperature during this deprotection step is typically held between 10°C and 35°C, preferably between 15°C and 20°C.

The reaction time is generally comprised between 2 and 10 hours, typically under stirring.

After the reaction is completed, an organic solvent is added. Suitable organic solvents are, for example, dichloromethane (DCM), ethyl acetate, chloroform, tetrahydrofuran (THF) or methyl *tert*-butyl ether (MTBE), among others. Preferably, the organic solvent is selected from dichloromethane and ethyl acetate, and more preferably is dichloromethane.

Acidic washing is performed at a pH value comprised in the range 0.5-6.0, preferably comprised in the range 1-3.5, preferably in the range 1.5-2.0. The pH may be adjusted, if needed, by adding an aqueous solution of sodium hydroxide or another suitable inorganic base.

In one embodiment, the separated organic phase is washed again with water or with a mixture of water and alcohol.

In one embodiment, the organic solvent in the resulting organic phase is subsequently distilled to obtain a residue.

The obtained residue may be dissolved again in an organic solvent, for example in dichloromethane and further distilled.

Optionally, after step c) there is a further purification step in which the product obtained in step c) is crystallized from a mixture of toluene and acetonitrile.

### Examples

### Example 1 Preparation of ticagrelor

### First step: preparation of compound of Formula II

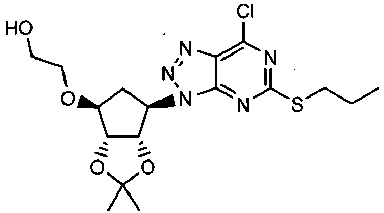

50 g (0.12 mol) of 2-[[(3aR,4S,6R,6aS)-6-[[5-amino-6-chloro-2-(propylthio)-4-pyrimidinyl]amino]tetrahydro-2,2-dimethyl-4H-cyclopenta-1,3-dioxol-4-yl]oxy]ethanol was dissolved in 275 ml of DCM and 58 g (0.97 mol) of acetic acid. The solution was cooled to 0-5°C and a solution of sodium nitrite (8.94 g, 0.13 mol) in water (26 ml) was added dropwise at a temperature of 0-5°C. The reaction mixture was stirred at 0-5°C for 2 hours. Afterwards, 250 ml of 15% brine was added and the reaction mixture was stirred at 0-10°C for 30 minutes. After phase separation, 150 ml of 15% brine was added to the organic phase and pH was corrected to 8.5-9.0 using 5% NaOH. After phase separation, the organic phase was washed with 250 ml of 15% brine at 0-10°C.

### Second step: preparation of compound of Formula IV

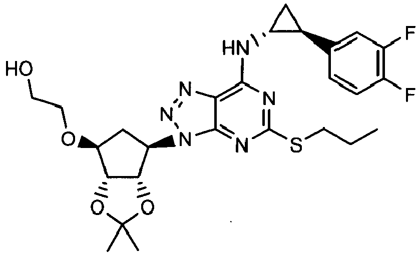

To the solution of 2-[[(3aR,4S,6R,6aS)-6-[7-chloro-5-(propylthio)-3H-1,2,3-triazolo[4,5-d]pyrimidin-3-yl]tetrahydro-2,2-dimethyl-4H-cyclopenta-1,3-dioxol-4-yl]oxy]ethanol in DCM obtained in the previous step, at 0-10°C, the mandelic acid salt of (trans-(1R,2S)-2-(3,4-difluorophenyl)cyclopropylamine) (38.92 g, 0.121 mol) and DIPEA (35.77 g, 0.277 mol) were added. The reaction mixture was heated to 20-25°C and stirred for 4h. Reaction mixture was then cooled to 10-20°C and washed with 335 ml of purified water. Next, 335 ml of purified water was added and pH was adjusted to 3.5-4.5 using 3.5% HCl solution at 10-20°C. Phases were separated, and the organic phase was washed with 335 ml of purified water. Solvents were distilled under reduced pressure and the compound of Formula IV was obtained as an oil residue.

### Third step: preparation of ticagrelor

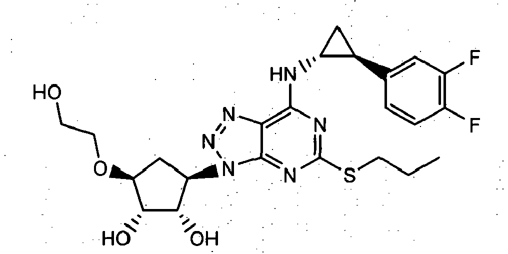

The residue obtained in the previous step was dissolved in 205 ml of methanol at 25-35°C. The solution was cooled to 10-15°C, and 56 ml of purified water were added, followed by addition of 56 ml of concentrated HCl. The reaction mixture was stirred for 4-7 hours at 16-20°C. Then 278 ml of DCM were added and the pH was corrected to 1.5-2.0 using 15% NaOH. After separation of phases, organic phase was washed with a mixture of 98 ml of methanol and 155 ml of purified water. The organic phase was washed again with a mixture of 65 ml of methanol and 155 ml of purified water. Solvents from the organic phase were distilled. The residue obtained after distillation was dissolved in 150 ml of DCM, the solvents were distilled again and solid ticagrelor was obtained.

The obtained ticagrelor was dissolved in 110 ml acetonitrile and 100 ml of toluene at 45-55°C. 5.76 g of silica gel were added and stirred for 20 minutes, the reaction mixture was then filtered, the filter was washed with a mixture of 12.6 ml of acetonitrile and of 25 ml of toluene. 550 ml of toluene were added to the filtrate arid the reaction mixture was then cooled to -5-5°C and stirred for 12 hours. The obtained ticagrelor was filtered and washed twice with a mixture of acetonitrile and toluene, and dried at 40-45°C to yield 50.5 g of ticagrelor (0.0966 mol, 81% mol yield). The obtained product was about 99.80%-99.85% HPLC pure.

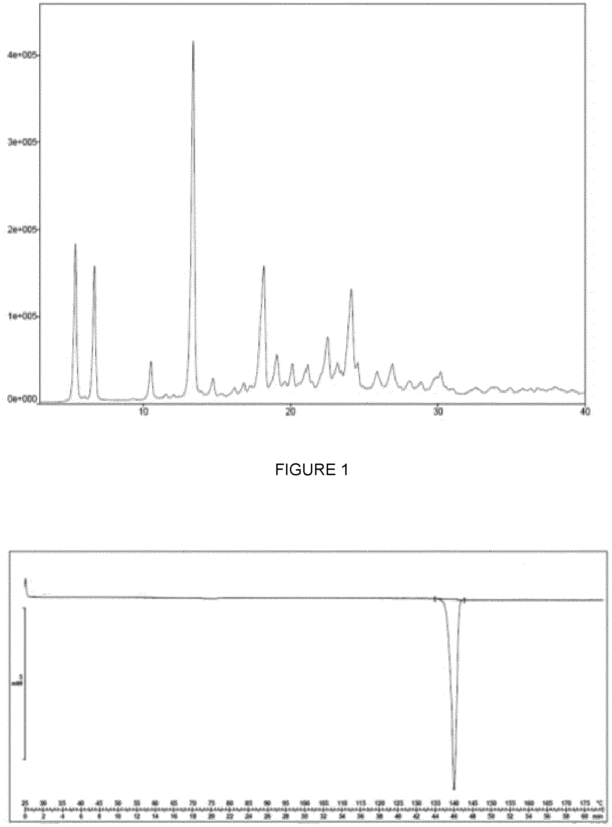

The XRPD and DSC of obtained ticagrelor are shown in Figure 1 and Figure 2.

### Example 2: Nitrosamine content of ticagrelor

Ticagrelor obtained in Example 1 was analyzed to determine the content of nitrosamines by GC-MS method (suitable methods for determining nitrosamines in active pharmaceutical ingredients are available, for example, at the Council of Europe website: https://www.edqm.eu/en/ad-hoc-projects-omcl-network).

In a comparative assay, ticagrelor was prepared according to the procedure disclosed in the prior art (WO01/92263-A1) and the nitrosamine content was also analyzed.

The comparative results are shown in the following table. NDEA means N-nitrosodiethylamine, NEIPA means *N*-nitrosoethylisopropylamine and NDIPA means N-nitrosodiisopropylamine. Prior art (A) means the product obtained after repetition of the procedure, exactly as disclosed in the prior art. Prior art (B) means repeating the prior art, but slightly increasing the amount of compound (III) (up to 1.03 mol per 1 mol of compound (I) and of TEA (20% more), in order to reach the minimum stoichiometric amounts required in the reaction, as they were used in an amount which was smaller, deficient amounts.

| | **Example 1** | **Prior art (A)** | **Prior art (B)** |
|---|---|---|---|
| NDEA | Below 0.0009 ppm (DL) | 0.026 ppm | 0.10 ppm |
| NEIPA | Below 0.0018 ppm (DL) | Below 0.0018 ppm (DL) | 0.01 ppm |
| NDIPA | Below 0.0018 ppm (DL) | Below 0.0018 ppm (DL) | Below 0.0018 ppm (DL) |
| Purity | 99.8% | 90.7% | 98.14% |
| Molar yield | 81% | 66% | 72% |

It can be observed that the procedure according to the invention provided the best yield of ticagrelor and the best purity of the compound. Furthermore, the process of the invention provided in all cases undetectable levels of *N*-nitrosamine impurities, while the in the ticagrelor obtained according to the prior art, higher levels were obtained.

## Claims

1. Process for the preparation of ticagrelor of formula: 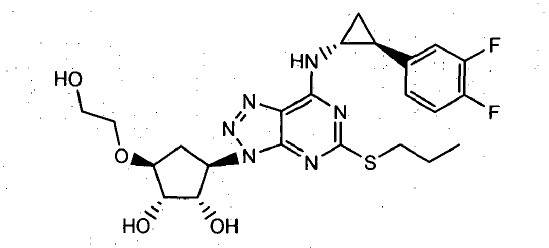 comprising the following steps:
a) reacting a compound of formula (I): 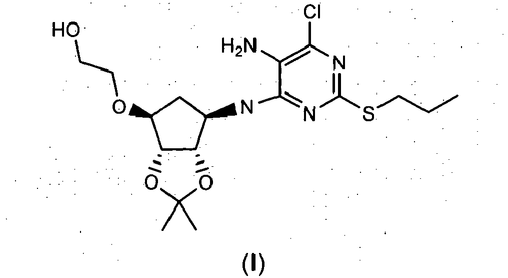 with sodium nitrite, in the presence of acetic acid to provide a compound of formula (II): 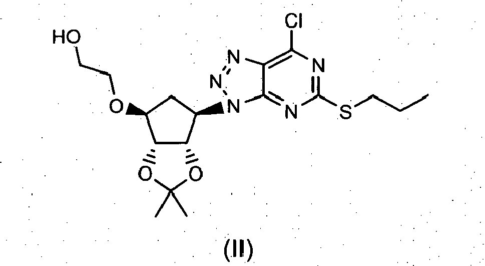 , wherein the reaction is followed by aqueous washing at a pH comprised in the range 2-6
b) reacting a compound of formula (II) with a compound of formula (III): 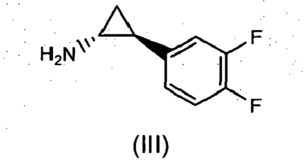 or a pharmaceutically acceptable salt thereof, in an organic solvent, in the presence of a base, to provide a compound of formula (IV): 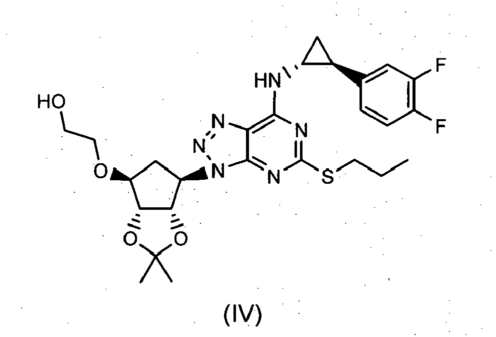 , wherein the reaction is followed by a first aqueous washing at a pH comprised in the range 7.5-12 and a second aqueous washing at a pH comprised in the range 2-6; and
c) deprotecting a compound of formula (IV) by treatment with an inorganic acid in a solvent mixture of water and a C₁-C₃ alcohol to provide ticagrelor of formula (I), wherein the deprotection step is followed by addition of an organic solvent and aqueous washing at a pH comprised in the range 0.5-6.0.

2. The process according to claim 1, characterized that the reaction of step a) is performed in a two-phase-system comprising water, acetic acid and an organic solvent.

3. The process according to claim 2, **characterized in that** the organic solvent is selected from the group consisting of dichloromethane (DCM), ethyl acetate, chloroform, tetrahydrofuran (THF) and methyl *tert*-butyl ether (MTBE), preferably is dichloromethane.

4. The process according to any one of claims 1 to 3, **characterized in that** the temperature during the reaction of step a) is held between 0°C and 10°C.

5. The process according to any one of claims 1 to 4, **characterized in that** in step a), after the first acidic aqueous washing, a second aqueous washing is performed at a pH comprised in the range 7.0-11.0, preferable comprised in the range 8.0-9.5.

6. The process according to any one of claims 1 to 5, **characterized in that** the organic solvent is step b) is selected from the group consisting of dichloromethane (DCM), ethyl acetate, chloroform, tetrahydrofuran (THF) and methyl *tert*-butyl ether (MTBE), preferably is dichloromethane.

7. The process according to any one of claims 1 to 6, **characterized in that** the base in step b) is an organic base which is selected from the group consisting of pyridine, pyrimidine, *N*-methylmorpholine, 1,4-diazabicyclo[2.2.2]octane (DABCO), N,N-dimethylaniline, *N,N*-diethylaniline, triethylamine (TEA), *N,N-*diethylisopropylamine, *N,N*-diisopropylethylamine (DIPEA), tripropylamine and tributylamine; preferably is selected from triethylamine and *N,N-*diisopropylethylamine, and more preferably is *N,N*-diisopropylethylamine.

8. The process according to any one of claims 1 to 7, **characterized in that** the temperature during the reaction of step b) is held between 15°C and 35°C.

9. The process according to any one of claims 1 to 8, **characterized in that** the organic solvent in step c) is selected from the group consisting of dichloromethane (DCM), ethyl acetate, chloroform, tetrahydrofuran (THF) and methyl *tert*-butyl ether (MTBE), preferably is dichloromethane.

10. The process according to any one of claims 1-9, **characterized in that** the inorganic acid in step c) is selected from hydrochloric acid, sulphuric acid and phosphoric acid, preferably is hydrochloric acid.

11. The process according to any one of claims 1 to 10, **characterized in that** the C₁-C₃ alcohol in step c) is selected from methanol, ethanol, propanol and isopropanol, preferably is selected from methanol, ethanol and isopropanol, and more preferably is methanol.

12. The process according to any one of claims 1 to 11, **characterized in that** the temperature during the deprotection reaction of step c) is held between 10°C and 35°C.

13. The process according to any one of claims 1 to 12, **characterized in that** ticagrelor obtained after step c) is crystallized from a mixture of toluene and acetonitrile.

14. Ticagrelor obtainable by the process of any one of claims 1 to 13, **characterized in that** the content of *N*-nitrosodiethylamine is below 0.0009 ppm, the content of N-nitrosoethylisopropylamine is below 0.0018 ppm and the content of *N-*nitrosodiisopropylamine is below 0.0018 ppm.
